# EUROPÄISCHE PATENTANMELDUNG

(11) **EP 2 532 354 A1**
(43) Veröffentlichungstag der Anmeldung: **12.12.2012**
(21) Anmeldenummer: 11169137.4
(22) Anmeldetag: 08.06.2011
(51) Int. Cl.: A61K 35/74, A61P 31/04

(54) **Sprühgetrocknete Lactobacillus Stämme / Zellen und deren Verwendung gegen Helicobacter Pylori**

(71) Anmelder: OrganoBalance GmbH, 13355 Berlin (DE)
(72) Erfinder: Arya, Stefanie, 13347 Berlin (DE); Goelling, Detlef, 25856 Hattstadt (DE); Holz, Caterina, 10405 Berlin (DE); Lang, Christiane, 14057 Berlin (DE)
(74) Vertreter: Simandi, Claus

(57) **Zusammenfassung**

Die Erfindung betrifft sprühgetrocknete Lactobacillus Stämme bzw. Lactobacillus Zellen (Milchsäurebakterien) sowie deren Verwendungen, insbesondere für pharmazeutische und/oder dietätische Zusammensetzungen zur Behandlung und Prophylaxe von Helicobacter pylori Infektionen an Mensch und Tier.

## Beschreibung

Die Erfindung betrifft sprühgetrocknete Lactobacillus Stämme bzw. Lactobacillus Zellen (Milchsäurebakterien) sowie deren Verwendungen, insbesondere für pharmazeutische und/oder dietätische Zusammensetzungen zur Behandlung und Prophylaxe von Helicobacter pylori Infektionen an Mensch und Tier.

Probiotische Mikroorganismen umfassen Zellen, welche vorteilhafte Wirkungen in menschlichen oder tierischen Körpern zeigen. Probiotische Zusammensetzungen enthalten solche Mikroorganismen. Vorteilhafte Wirkungen können insbesondere in der Verbesserung der Mikroflora des Verdauungstraktes bestehen. Insbesondere können in der Mikroflora unerwünschte andere Mikroorganismen durch unmittelbare Wechselwirkungen zwischen den probiotischen Mikroorganismen und den unerwünschten Mikroorganismen, durch mittelbare Wechselwirkungen auf Grund von Hemmungen des Metabolismus des unerwünschten Mikroorganismus durch Expressionsprodukte des probiotischen Mikroorganismus, oder durch Verstärkung des natürlichen Immunsystems gehemmt werden. Allgemein wird angenommen, dass ein Hauptmechanismus die kompetitive Besiedlung des Gastrointestinaltraktes ein wesentliches Wirkelement ist, wodurch unerwünschte Mikroorganismen die Schleimhaut (Mucosa) nicht mehr in störendem Maße besiedeln können bzw. verdrängt werden.

Eine Gruppe probiotischer Mikroorganismen wird beispielsweise durch Laktobazillenstämme gebildet. Hierbei handelt es sich typischerweise um gram-positive, mikroaerophile oder anaerobe Bakterien, welche Zucker fermentieren unter Bildung von Säuren, insbesondere von Milchsäure.

Aus US 5,716,615 ist eine pharmazeutische Zusammensetzung bekannt, welche unter anderem Laktobazillen enthält. Diese ist unter anderem einsetzbar zur Behandlung von Erkrankungen des Gastrointestinaltraktes.

Aus WO 2004/087891 sind Lactobacillus Stämme bekannt, welche zur Herstellung von pharmazeutischen oder dietätischen Zusammensetzungen zur Behandlung von Infektionen des Gastrointestinaltraktes mit Helicobacter pylori geeignet sind.

Wechselwirkungen von Laktobazillen mit Helicobacter pylori sind u. a. beschrieben in Wang et al., Am. J. Clin. Nutr. 80:737-41 (2004), Felley et al., Best Practice & Research Clinical Gastroenterology 17 (5): 785-791 (2003), Cazzato et al., Scandinavian Journal of Nutrition 48(1): 26-31 (2004) und Sgouras et al., Applied and Environmental Microbiology 70 (1): 518-526 (2004).

Helicobacter pylori ist eine spiralförmige Bakterie, die den Magen kolonisiert, wobei mittels Produktion von Urease der pH Wert im Magen angehoben und in dieser Weise die Bakterien vor der Magensäure geschützt werden. Die Bakterien penetrieren in die Schleimhaut und lagern sich an den Epithelzellen an. Eine solche Infektion aktiviert das körpereigene Immunsystem, wobei die Immunantwort jedoch nicht hinreichend effektiv zur Beseitigung der Infektion ist, mit der Folge einer sich verstärkenden Immunantwort, die zu einer chronischen Entzündung und Erkrankung wie Gastritis bzw. Magengeschwüren und schließlich zum Karzinom führt.

Wenn Zellen untereinander binden und Agglomerate bilden, bezeichnet man diesen Vorgang als Aggregation. Wenn bei dieser Aggregat-Bildung nur eine Zellart beteiligt ist, wird das als Autoaggregation oder Selbstaggregation bezeichnet. Sind bei der Aggregat-Bildung mindestens zwei verschiedene Zellarten beteiligt, bezeichnet man den Vorgang als Co-Aggregation.WO 2007/073709 der Anmelderin beschreibt Co-Aggregate von Laktobazillen mit Helicobacter pylori, welche zur Prophylaxe, Behandlung und/oder einer Eradikationtherapie von Helicobacter pylori Infektionen genutzt werden können, insbesondere wird zumindest eine Reduktion von Helicobacter pylori erreicht. Ferner werden in WO 2007/073709 hierzu geeignete Lactobacillus Stämme beschrieben, die an der Deutschen Sammlung von Mikroorganismen und Zellkulturen GmbH (DSMZ), Mascheroder Weg 1b, D-38124 Braunschweig, Deutschland, hinterlegt wurden und zwar: DSM 17646, DSM 17647, DSM 17648, DSM 17649, DSM 17650, DSM 17651, DSM 17652 und DSM 17653.

Die geeigneten Lactobacillus Zellen bilden beim Kontakt mit Helicobacter pylori Zellen Co-Aggregate. Durch die Bildung von Co-Aggregaten wird Helicobacter pylori daran gehindert, in die Magenschleimhaut einzudringen. Die Helicobacter pylori Zellen, insbesondere deren Zelloberfläche, werden durch die Lactobacillus Zellen maskiert, so dass die Helicobacter pylori Zellen nicht mehr in der Lage sind, an die Magenepithelzellen zu binden. Durch die verhinderte Bindung von Helicobacter pylori an die Magenepithelzellen bleiben Entzündungsreaktionen aus. Die maskierten und damit deaktivierten Helicobacter pylori Zellen werden in Form von Co-Aggregaten durch den Magen-Darm-Trakt geschleust und ausgeschieden. Es ist also möglich, durch die Gabe von geeigneten Lactobacillus Zellen die Helicobacter pylori Zellen im Magen zu reduzieren bzw. zu eradizieren. Die Anwendung der Lactobacillus Zellen kann prophylaktisch oder kurativ erfolgen. Allerdings gilt es, diesen Therapieansatz zu verbessern.

Daher liegt der Erfindung die Aufgabe zugrunde, ein verbessertes Verfahren zur Prophylaxe und Behandlung von Helicobacter pylori Infektionen bereitzustellen. Insbesondere ein solches Verfahren, das eine verbesserte Co-Aggregatbildung erlaubt, wobei mittels ausgebildeten Co-Aggregaten die Besiedlung der Magenschleimhaut mit Helicobacter pylori deutlich besser inhibiert/verringert wird.

Der Einsatz und die Herstellung von sprühgetrockneten Laktobazillen im Stand der Technik ist bekannt, z. B. Gardiner et al., Comparative Survival Rates of Human-Derived Probiotic Lactobacillus paracasei and L. salivarius Strains during Heat Treatment and Spray Drying, Applied and Enviromental Microbiology 66 (6): 2605-2612 (2000) und Teixeira et al., Survival of Lactobacillus delbrueckii ssp. Bulgaricus Following Spray-Drying, J. Dairy Sci 78:1025-1031 (1995).

Nicht beschrieben ist jedoch die Verwendung von sprühgetrockneten Laktobazillen zur Bildung von Co-Aggregaten mit Helicobacter pylori und deren besonderen Eignung in Form einer pharmazeutischen oder diätischen Zusammensetzung.

Überraschender Weise kann eine verbesserte Verhinderung und Prophylaxe von Helicobacter pylori Infektionen erzielt werden, falls sprühgetrocknete Lactobacillus Zellen zur Co-Aggregation eingesetzt werden.

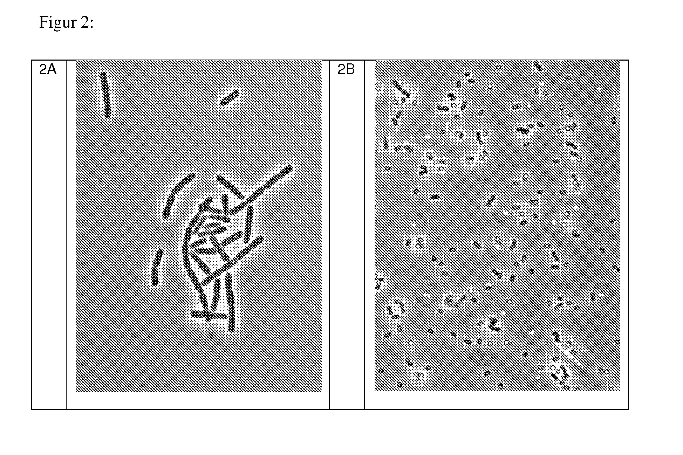

Sprühgetrocknete Lactobacillus Zellen können besonders vorteilhaft große Co-Aggregate mit Helicobacter pylori bilden und auf diese Weise eine effiziente Reduzierung der Keimbelastung durch Helicobacter pylori bewirken. Besonders vorteilhaft führt die Sprühtrocknung der Lactobacillus Zellen zu einer Verkleinerung der Lactobacillus Zellen, wobei zudem eine Vereinzelung / Separation der Zellen erfolgt. Im Gegensatz zu anderen Trocknungsverfahren oder ohne Trocknung durchgeführten Verfahren werden keine Ketten von zwei bis zehn Lactobacillus Zellen erhalten (Figur 2A), sondern - für die Sprühtrocknung charakteristisch - einzelne Zellen (Mono) oder Zweierzellen (Dimer) (Figur 2B). Diese sprühgetrockneten "Keimzellen" eignen sich hervorragend zur in-situ Bildung der Co-Aggregate nach Applikation im Magenmedium und es werden vorteilhafte hochdichte, kompakte und effiziente Co-Aggregate aus Lactobacillus Zellen und Helicobacter pylori (Zellen) erhalten, die zudem eine vorteilhafte geringe sterische Hinderung in der Bildungsphase der Co-Aggregate aufweisen. Solche erfindungsgemäßen sprühgetrockneten Lactobacillus Zellen weisen eine höhere Bindungsaffinität an Helicobacter pylori auf, als beispielsweise jene die gemäß der technischen Lehre aus WO 2007/073709 hergestellt werden können. Durch die höhere Bindungsaffinität können durch weniger Laktobazillen Zellen mehr Helicobacter pylori Zellen maskiert und gebunden werden. Die höhere Bindungsaffinität führt weiterhin zu einer höheren Stabilität der gebildeten Co-Aggregate.

Weiterhin ist vorteilhaft, dass sprühgetrocknete Lactobacillus Zellen ebenfalls einen erhöhten Anteil an nicht-lebenden und / oder Fragmenten von Lactobacillus Zellen aufweisen, welche ebenfalls die spontane Co-Aggregat Bildung unterstützen.

Die erfindungsgemäßen Co-Aggregate durchlaufen den Gastrointestinaltrakt und verlassen den Körper auf natürlichem Wege. Selbst bei bereits erfolgter Infektion ist dieser Wirkmechanismus erfindungsgemäßer sprühgetrockneter Lactobacillus Stämme hilfreich, da eine weitere Infektion mit zusätzlichen Helicobacter pylori Bakterien verhindert wird und so die bestehende Infektion durch Inaktivierung / Ausscheidung der vorhandenen Helicobacter pylori Bakterien leichter bekämpft werden kann. Hinzu kommt, dass erfindungsgemäße Lactobacillus Stämme vermutlich auch zur Hemmung der Urease Aktivität von Helicobacter pylori in der Lage sind, so dass die Helicobacter pylori Bakterien in den Co-Aggregaten ihren Schutz gegen den Angriff von Magensäure verlieren. Insofern wird auch ein synergistischer Effekt erzielt.

Sprühgetrocknete Lactobacillus Stämme oder Lactobacillus Zellen weisen den besonderen Vorteil auf, dass die Bindungsaffinität zu Helicobacter pylori erhöht ist (supra). Weil die Oberfläche von Helicobacter pylori durch die Lactobacillus Zellen maskiert ist, ist eine Penetration von Helicobacter pylori in die Schleimhaut verhindert und folglich kann eine Helicobacter pylori Infektion samt chronischen Entzündungsprozessen nicht eintreten und Folgeerkrankungen wie Gastritis, Magengeschwüre oder Ulcus bis hin zum Magenkrebs werden sicher verhindert.

Darüber hinaus weisen die erfindungsgemäßen Co-Aggregate aus sprühgetrockneten Lactobacillus Zellen und Helicobacter pylori Zellen eine erhöhte Stabilität auf, so dass erstens eine erhöhte Zahl von Helicobacter pylori in diese Co-Aggregate eingebunden werden kann und zweitens keine Wiederfreisetzung von vorher gebundenen Helicobacter pylori Zellen durch z. B. Magenbewegungen stattfindet. Dieser Vorteil erlaubt gegenüber den im Stand der Technik bekannten Co-Aggregaten eine vorteilhafte niedrigere Dosierung. Ferner erlauben die erfindungsgemäßen sprühgetrockneten Lactobacillus Zellen eine vorteilhafte erhöhte Löslichkeit in Wasser, so dass eine verbesserte Verteilung der sprühgetrockneten Lactobacillus Zellen und der erhaltenen Co-Aggregate im Magenraum erreicht werden kann.

Ferner konnten die Erfinder feststellen, dass durch die Sprühtrocknung der Lactobacillus Zellen eine unkontrollierte Aggregatbildung der Laktobazillen selbst (Autoaggregation) verhindert oder gegenüber frischen nicht-sprühgetrockneten Lactobazillen zumindest stark reduziert ist. Durch Autoaggregation der Lactobacillus Zellen untereinander werden die Bindungsstellen für die Maskierung von Helicobacter pylori besetzt. Die Reduzierung bzw. Verhinderung der Autoaggregation führt also ebenfalls zu einer vorteilhaften niedrigeren Dosierung der eingesetzten Lactobazillen gegenüber dem Stand der Technik.

Das erfindungsgemäße Sprühverfahren bewirkt eine morphologische Änderung der Laktobazillen, so dass die Bindungsaffinität für Helicobacter pylori erhöht ist und damit eine verbesserte Co-Aggregatbildung erfolgen kann.

Daher betrifft die Erfindung eine pharmazeutische oder diätische Zusammensetzung enthaltend sprühgetrocknete Lactobacillus Zellen zur Prophylaxe und Behandlung von Helicobacter pylori Infektionen an Mensch und Tier, insbesondere Säugetier.

In einer weiteren Ausführungsform der Erfindung liegen die sprühgetrockneten Lactobacillus Zellen in der Zusammensetzung vorzugsweise und im Wesentlichen in einer mono und /oder dimeren Form vor (supra, Fig. 2B).

Weiterhin betrifft die Erfindung eine solche Zusammensetzung, wobei nach Applikation an Mensch oder Tier Co-Aggregate mit Helicobacter pylori im Magenmedium in-situ gebildet werden, die vorzugsweise größer und nicht kleiner als 50 µm, insbesondere größer und nicht kleiner als 100 µm, 150 µm, insbesondere größer als 500 µm, besonders bevorzugt größer als 1.000 µm oder 1.100 µm sind.

Der Begriff "Lactobacillus Zellen" iSd. Erfindung (iwS. Milchsäurebakterien, auch Lactobazillen) umfasst solche Mikroorganismen, die Kohlenhydrate, insbesondere Glukose und Lactose, zur Milchsäurevergärung benötigen und zumeist den Embden-Meyerhof-Biosyntheseweg nutzen. Die Lactobacillus Zellen werden taxonomisch in der Familie Lactobacteriaceae zusammengefasst. Sie sind gram-positiv, nicht sporenbildend und im Allgemeinen unbeweglich. Die Lactobacillus Zellen leben anaerob, sind jedoch aerotolerant, obwohl sie keine Hämine (Cytochrom, Katalase) enthalten (Schleifer et al., System. Appl. Microb.: 18, 461-467 (1995) oder Ludwiq et al., System. Appl. Microb. 15: 487-501 (1992). Erfindungsgemäß umfasst sind insbesondere solche Spezies, die für eine homofermentative Milchsäuregärung oder heterofermentative Milchsäuregärung geeignet sind. Weiterhin bevorzugt sind solche Lactobacillus Zellen ausgewählt aus der Gruppe *Lactobacillus lactis, Lactobacillus helveticus, Lactobacillus acidophilus, Lactobacillus bulgaricus, Lactobacillus delbrueckii, Lactobacillus casei, Lactobacillus paracasei, Lactobacillus pentosus, Lactobacillus rhamnosus* und *Lactobacillus plantarum* (alle homofermentativ), weiterhin *Lactobacillus brevis, Lactobacillus fermentum, Lactobacillus viridescens* als auch *Bifidobacterium bifidum* (alle heterofermentativ). Beispielhafte geeignete Lactobacillus Zellen der Anmelderin sind hinterlegt: DSM 17646, DSM 17647, 'DSM 17648, DSM 17649, DSM 17650, DSM 17651, DSM 17652 und DSM 17653 (supra). *Lactobacillus fermentum, Lactobacillus brevis* und *Lactobacillus pentosus* sind erfindungsgemäß bevorzugt.

Erfindungsgemäß umfasst sind ebenfalls solche "Lactobacillus Zellen", die inaktiviert, lebend oder nicht-lebend sind oder Teile und Fragmente, z.B. enzymatische oder mechanische Spaltprodukte (z.B. French Press etc.), von Lactobacillus Zellen darstellen soweit diese die Eignung zur Co-Aggregation aufweisen.

All diese Mikroorganismen werden nach- und vorstehend "Lactobacillus Zellen" genannt.

Die wesentlichen Kulturbedingungen des menschlichen Magentraktes umfassen einen pH-Wert im Bereich von 1,8 bis 4,5 und die Gegenwart von Pepsin sowie NaCl. Ein Referenzmedium, welches für solche Kulturbedingungen charakteristisch ist, besteht aus den folgenden Komponenten: Wasser, 5 g/l NaCl sowie 3 g/l Pepsin wobei der pH Wert auf 2,0 bzw. 4,0 mit Salzsäure eingestellt ist, um einen leeren bzw. gefüllten Magen zu simulieren.

Der Begriff der "Co-Aggregation" iSd. Erfindung bezeichnet die Bildung von Zellaggregaten einer Größe von zumindest 50 µm oder 100 µm und mehr, enthaltend erfindungsgemäße sprühgetrocknete Lactobacillus Zellen und Helicobacter pylori Zellen, in Suspensionen, beispielsweise gemäß der folgenden Beispiele, insbesondere in einem Referenzmedium, wie vorstehend beschrieben.

Der Begriff "sprühgetrocknete Lactobacillus Zellen" iSd. Erfindung bedeutet, dass die Lactobacillus Zellen mittels einem Sprühtrocknungs- oder Zerstäubungsverfahren(Synonym) getrocknet werden, wobei z.B. eine Suspension von Lactobacillus Zellen in feine nebelartige Tröpfchen zerteilt wird und ein Pulver erhalten werden kann.

Bei einer erfindungsgemäßen Sprühtrocknung wird eine Lösung oder Suspension enthaltend Lactobacillus Zellen in ein heißes Trocknungsmedium versprüht und dadurch getrocknet. Das zu versprühende Gemisch kann in Form von Lösung, Emulsion, Suspension oder Dispersion vorliegen. Es wird mit Hilfe einer Düse oder einem Sprührad in Millionen von einzelnen Tröpfchen zerstäubt, wobei die Oberfläche stark vergrößert wird. Das Lösungsmittel, wie Wasser, wird sofort durch die heiße Luft verdampft und abgeführt.

Das Sprühtrocknungs- oder Zerstäubungsverfahren kann von anderen Trocknungsverfahren unterschieden werden, da der Einsatz einer Düse oder gleichwirkendes Mittel erforderlich ist, solche wie Einstoffdüse, Hohlkegeldüse, Druckdüse, Zweistoffdüse außenmischend, pneumatische Düse, Zweistoffdüse innenmischend, Zerstäuberscheibe oder Ultraschallzerstäuber.

Sprühtrocknungsverfahren sind im Stand der Technik beschrieben und dem Fachmann geläufig (siehe Gardiner et al., Teixeira et al. (supra) oder EP74050 und EP285682). Geräte sind einschlägig bekannt und beschrieben, wie z.B. Mini Sprühtrockner B-191 oder B-290 der Firma Büchi Labortechnik AG (Deutschland) oder SD-6.3-R der Firma GEA Niro (Dänemark). Ferner ist bekannt, dass beliebige Hilfs- und Zusatzstoffe verwendet werden können.

Die Erfindung betrifft des Weiteren eine pharmazeutische und/oder dietätische Zusammensetzung enthaltend eine physiologisch wirksame Dosis an erfindungsgemäßen sprühgetrockneten Lactobacillus Zellen sowie einen physiologisch verträglichen Träger. Bei pharmazeutischen Zusammensetzungen handelt es sich um Zusammensetzungen, die einzig therapeutischen oder prophylaktischen Zwecken dienen und wobei neben den Lactobacillus Zellen lediglich in der Galenik übliche Hilfs- und/oder Trägerstoffe zugegen sind. Bei dietätischen Zusammensetzungen im Sinne der vorliegenden Erfindung handelt es sich um Zusammensetzungen, welche neben den erfindungsgemäßen sprühgetrockneten Lactobacillus Zellen ein Nahrungs- oder Lebensmittel (siehe z.B. nicht abschließend EU-Richtlinie 2002/46/EG vom 10. Juni 2002) und/oder ein Futtermittel für Haus- und/oder Nutztiere und / oder Nahrungsergänzungsmittel umfassen, ggfs. enthaltend Hilfs- und Zusatzstoffe.

Die Erfindung betrifft auch die Verwendung von erfindungsgemäßen sprühgetrockneten Lactobacillus Zellen zur Herstellung einer pharmazeutischen oder dietätischen Zusammensetzung, insbesondere zur Prophylaxe und/oder Behandlung von durch Infektion mit Helicobacter pylori bedingten Erkrankungen, beispielsweise Gastrointestinalerkrankungen. Hierzu zählen insbesondere Gastritis, Magengeschwüre, Ulcus und Magenkrebs.

Weiterhin betrifft die Erfindung die Verwendung der erfindungsgemäßen sprühgetrockneten Lactobacillus Zellen zur Eradikation bzw. Eradikationstherapie von Helicobacter pylori, ggfs. in Kombination mit weiteren geeigneten Wirkstoffen, wie Antibiotika.

Eine erfindungsgemäße pharmazeutische oder diätische Zusammensetzung kann dadurch gekennzeichnet sein, dass sie 10² bis 10¹⁵, vorzugsweise 10⁴ oder 10⁸ bis 10¹², insbesondere 10⁸ bis 10¹⁰, sprühgetrocknete Lactobacillus Zellen enthält. Bezugsgröße ist dabei eine Gabeeinheit, beispielsweise eine Tablette. Vorzugsweise ist die Zusammensetzung zur oralen Gabe hergerichtet.

Die galenische Herrichtung einer erfindungsgemäßen pharmazeutischen oder diätischen Zusammensetzung kann in fachüblicher Weise erfolgen. Geeignete feste oder flüssige galenische Zubereitungsformen sind beispielsweise Granulate, Pulver, Dragees, Tabletten, (Mikro-) Kapseln, Hartkapseln, Suppositorien, Sirupe, Säfte, Suspensionen, oder Emulsionen, bei deren Herstellung übliche Hilfsmittel wie Trägerstoffe, Spreng-, Binde-, Überzugs-, Quellungs-, Gleit^{TM} oder Schmiermittel, Geschmacksstoffe, Süßungsmittel und Lösungsvermittler, Verwendung finden. Als Hilfsstoffe sei Magnesium-Stearat, Natriumchlorid, Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass Zellen mindestens eines erfindungsgemäß verwendeten Lactobacillus Stammes in definierter Dosis mit einem pharmazeutisch geeigneten und physiologisch verträglichen Träger und ggf. weiteren geeigneten Wirk-, Zusatz- oder Hilfsstoffen mit definierter Dosis gemischt und zu der gewünschten Darreichungsform hergerichtet ist. Als Träger kommen insbesondere Stoffe in Frage, die ausgewählt sind aus der Gruppe bestehend aus Maltodextrin, mikrokristalline Zellulose, Stärke, insbesondere Maisstärke, Levulose, Lactose, Dextrose, und Mischungen solcher Substanzen. Die Zusammensetzung kann 0,1 bis 95 Gew.-% Träger und 5 bis 99.9 Gew.-% sprühgetrocknete Lactobacillus Zellen, bezogen auf die Gesamtmenge an Zellen und Träger, enthalten bzw. hieraus bestehen.

Im Falle der dietätischen Zusammensetzung kann vorgesehen sein, dass die Zusammensetzung 10² bis 10¹⁵, vorzugsweise 10⁴ bis 10¹², insbesondere 10⁸ bis 10¹⁰, Lactobacillus Zellen enthält. Bezugsgröße ist eine Gabeeinheit, beispielsweise eine Verpackungseinheit eines Lebensmittels zum Verkauf an einen Endverbraucher. Der physiologisch verträgliche Träger wird in der Regel ein Lebensmittel sein, welches insbesondere ausgewählt ist aus der Gruppe bestehend aus Milchprodukten, fermentierten Milchprodukten, Milch, Joghurt, Käse, Cerealien, Müsliriegel, Backwaren- und Getränken und Kindernahrungszubereitungen. Geeignete erfindungsgemäße Nahrungs- oder Lebensmittel, einschließlich Wasser, sind solche wie z.B. in der Verordnung (EG) Nr. 178/2002 vom 28. Januar 2002 nicht abschließend definiert.

Die Erfindung betrifft weiterhin ein Verfahren zur Herstellung einer erfindungsgemäßen pharmazeutischen und/oder dietätischen Zusammensetzung, wobei die sprühgetrockneten Lactobacillus Zellen mit dem physiologisch verträglichen Träger gemischt und vorzugsweise zur oralen Gabe hergerichtet werden.

Ferner betrifft die Erfindung ein Verfahren zur Herstellung einer erfindungsgemäßen Zusammensetzung, wobei die Lactobacillus Zellen (i) optional angeimft und angereichert (ii) fermentiert und (iii) sprühgetrocknet werden und anschließend mit einem physiologisch verträglichen Träger gemischt und vorzugsweise zur oralen Gabe hergerichtet werden.

Schließlich betrifft die Erfindung ein Verfahren zur Prophylaxe oder Behandlung eines Menschen oder Tieres, insbesondere Patient oder Proband, welche an einer durch eine Helicobacter pylori Infektion verursachten Erkrankung, insbesondere Gastritis bzw. Magengeschwür, leidet oder hieran zu erkranken droht, wobei jene Person eine physiologisch wirksame Dosis einer erfindungsgemäßen pharmazeutischen und/oder dietätischen Zusammensetzung ein- bis fünfmal täglich dargereicht wird. Die Gabe kann über einen zeitlich beschränkten Zeitraum, beispielsweise 1 bis 30 Wochen, erfolgen, oder zeitlich unbeschränkt. Insbesondere letzteres eignet sich für eine dauerhafte Prophylaxe sowie eine Vorbeuge gegen Rückfallerkrankungen.

Im Folgenden wird die Erfindung anhand von lediglich Ausführungsformen darstellenden Beispielen näher erläutert, ohne die Erfindung auf diese Beispiele beschränken zu wollen.

### Beispiele und Figuren:

### Beispiel 1: Lagerung verwendeter Stämme

Die Lagerung der Lactobacillus Stämme erfolgte im gefrorenen Zustand. 1 ml einer bis zur stationären Phase (OD₆₀₀/ml 4-8) in MRS Medium (55g/l, pH 6,5; Difco, USA) kultivierten Kultur wurde mit 500 µl einer 50 % igen (v/v) sterilen Glycerinlösung gemischt und die Mischung bei — 80 °C eingefroren.

Die Lagerung von Helicobacter pylori erfolgte in gefrorenem Zustand. 1 ml einer bis zur stationären Phase in Brucella broth (28g/l, pH 7,0; BD, USA), supplementiert mit 10 % (v/v) fetalem Kälberserum (Biochrom) , kultivierten Kultur wurde mit 500 µl einer 50 % igen (v/v) sterilen Glycerinlösung gemischt und die Mischung bei - 80 °C eingefroren.

### Beispiel 2: Prozess zur Herstellung von sprühgetrockneten Lactobacillus Zellen

Ein sprühgetrockneter Lactobacillus, der in der Lage ist, Helicobacter pylori zu coaggregieren, unter Kulturbedingungen des menschlichen Verdauungstraktes, insbesondere des Magens, wurde wie im folgenden beschrieben hergestellt:

Die Vorkultur 1 fand in einem 15 ml Reaktionsgefäß in 10 ml MRS-Medium statt. Das Medium wurde mit frisch kultivierten, tiefgefrorenen, gefriergetrockneten oder sprühgetrockneten Zellen des Lactobacillus Stamm angeimpft. Diese Vorkultur 1 wurde für 24 h bei 37 °C unter anaeroben Bedingungen inkubiert. Die komplette Vorkultur 1 wurde zum Animpfen der Vorkultur 2 verwendet. Vorkultur 2 bestand aus 240 ml MRS-Medium mit 10 ml der Vorkultur 1. Vorkultur 2 wurde für 19 h bei 37 °C unter anaeroben Bedingungen inkubiert. Am Ende jeder Vorkultur wurden optische Dichte, pH-Wert und cfu bestimmt.

Für die Fermentation von Lactobacillus wurden 5 Liter MRS verwendet. Der Fermenter mit allen erforderlichen Komponenten wurde autoklaviert. Sollwerte für Temperatur und Rührerdrehzahl waren 37 °C und 150 U / min. Die Animpfkonzentration betrug 5%. Am Ende der Fermentation wurden Zellzahl, Kolonie bildende Einheiten, pH, optische Dichte bei 600 nm und die Konzentration von Glukose und Laktat bestimmt. Im Anschluss wurde die Fermentationsbrühe 10fach bis 20fach aufkonzentriert. Im Anschluss wurde das Konzentrat eingefroren.

Vor der Trocknung wurde das 20-fach-Konzentrat aufgetaut und bei 4300 x g für 15 Minuten zentrifugiert. Die Zellen wurden einmal mit 0,9 % NaCl-Lösung gewaschen und bei 4300 x g für 15 Minuten erneut zentrifugiert. Dann wurden die Zellen in 500 ml 10 % NaCl-Lösung resuspendiert.

Die Sprühtrocknung wurde in einem Büchi Mini Spray Dryer B-191 durchgeführt. Die Eingangstemperatur betrug 140 °C. Die Ausgangstemperatur betrug 86 °C. Der Heißluftstrom betrug 550 L/h. Die Aspirator-Leistung betrug 75 %, die Pumprate 5 %. Die Zellsuspension wurde mit vorstehenden Parametern getrocknet und im Anschluss wurde das sprühgetrocknete Lactobacillus Pulver entnommen.

Daraufhin folgte eine Bestimmung der Gesamtzellzahl sowie Lebendzellzahl des Lactobacillus Stamms pro Gramm sprühgetrocknetem Pulver. Die mikroskopische Untersuchung des sprühgetrockneten Pulvers (Figur 2B) zeigte, dass die Lactobacillus Zellen kleiner sind als nicht-sprühgetrocknete Lactobacillus Zellen (Figur 2A) und dass die sprühgetrockneten Lactobacillus Zellen in Mono- und Dimeren vorliegen, während die nicht-sprühgetrockneten Lactobacillus Zellen längere Ketten aufweisen. Bis zur weiteren Verwendung wurden die sprühgetrockneten Lactobacillus Zellen bei 4 °C gelagert.

### Beispiel 3: Vergleich der Co-Aggregation von Helicobacter pylori durch nicht sprühgetrocknete und sprühgetrocknete Lactobacillus Stämme bzw. Lactobacillus Zellen unter Magenbedingungen

Die Kultivierung der Lactobacillus Zellen erfolgte in geschlossenen 15 ml Röhrchen in MRS Medium bei 37 °C für 24 h. Für die Untersuchung von sprühgetrockneten Laktobazillen wurden das sprühgetrocknete Pulver in PBS mit einer Konzentration von 10 mg / ml resuspendiert. Helicobacter pylori wurde für ca. 2 Tage in Erlenmeyerkolben unter mikroaerophilen Bedingungen in Brucella Bouillon (28 g/l, pH 7,0; BD, USA) mit 10% fötalem Kälberserum (Biochrom) bei 37 °C kultiviert. Nach der Kultivierung wurde die Zellmorphologie von Helicobacter pylori mikroskopisch untersucht. Es wurden Assays mit Zellen sigmoidaler Morphologie oder mit Zellen coccoider Morphologie durchgeführt. Auch Kulturen mit gemischter Morphologie wurden untersucht.

Die jeweiligen Zellen wurden durch Zentrifugation bei 3200 g für 10 min. geerntet und der Überstand wurde verworfen. Die Lactobacillus Zellen wurden einmal in 5 ml Puffer gewaschen und in 5 ml PBS Puffer resuspendiert (PBS-Puffer enthaltend 1,5 g/l Na₂HPO₄*2H₂O, 0,2 g/l KH₂PO₄ und 8,8 g/l NaCl). Die Helicobacter pylori Zellen wurden einmal in 5 ml PBS Puffer gewaschen und in 5 ml künstlichem Magensaft resuspendiert (enthaltend 5 g/l NaCl und 3 g/l Pepsin (Sigma)). Der OD₆₀₀ Wert wurde für die jeweiligen Zellen gemessen und auf einen Wert von 2 für Helicobacter pylori bzw. 4 für Lactobacillus durch Zugabe von künstlichem Magensaft bzw. PBS Puffer eingestellt.

2,5 ml jeder so erhaltenen Zellsuspension (Helicobacter pylori/Lactobacillus) wurden gemischt und die Mischung wurde für 10 s bis 10 min. geschüttelt. Das Ergebnis war sowohl optisch sichtbar durch deutliche Flockulation in den Proben, die Helicobacter pylori und Lactobacillus enthielten, und wurde auch mikroskopisch untersucht.

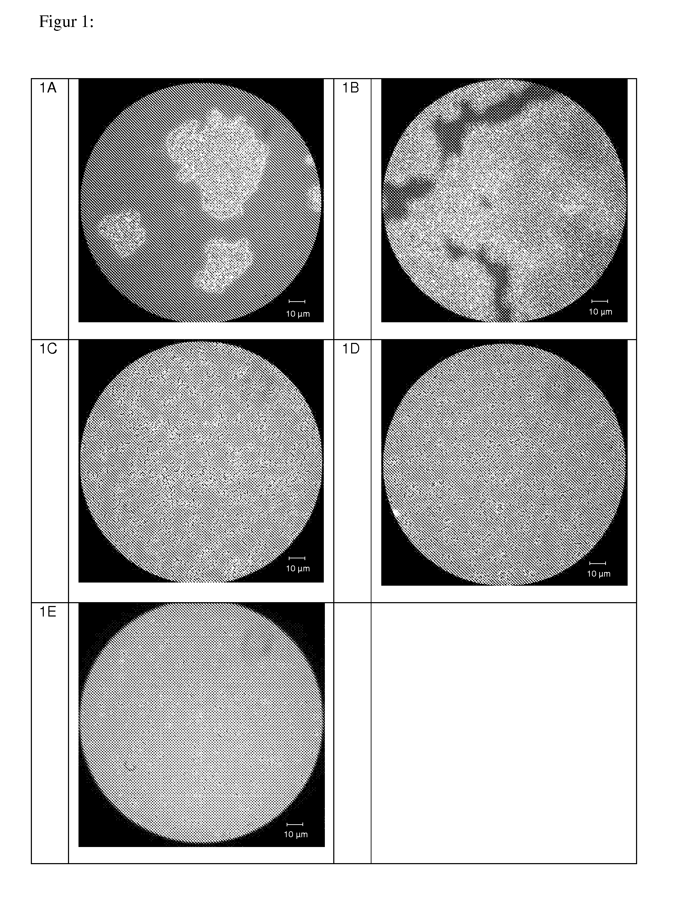

Figur 1A zeigt das mikroskopische Bild von Co-Aggregaten aus Helicobacter pylori und nicht-sprühgetrockneten Lactobacillus DSM 17648 Zellen. Figur 1B zeigt das mikroskopische Bild von Co-Aggregaten aus Helicobacter pylori und sprühgetrockneten Lactobacillus DSM 17648 Zellen. Diese Co-Aggregate sind deutlich größer als die Co-Aggregate mit nicht-sprühgetrockneten Lactobacillus Zellen. Kontrollexperimente auf Selbstaggregation wurden durch separate Untersuchung von Kulturen mit jeweils Lactobacillus und Helicobacter pylori allein durchgeführt. In den Figuren 1C, 1D und 1E ist weder Co-Aggregation noch Autoaggregation zu sehen. Figur 1C zeigt das mikroskopische Bild von frisch kultivierten Zellen von Lactobacillus DSM 17648 unter künstlichen Magenbedingungen. Figur 1D zeigt das mikroskopische Bild von sprühgetrockneten Zellen von Lactobacillus DSM 17648 unter künstlichen Magenbedingungen. Figur 1E zeigt das mikroskopische Bild von frisch kultivierten Helicobacter pylori Zellen unter künstlichen Magenbedingungen.

### Beispiel 4: Testen der Stabilität der Co-Aggregate zwischen sprühgetrockneten Lactobacillus Zellen und Helicobacter pylori Zellen

Zum Testen der Stabilität der Co-Aggregate unter in vivo Bedingungen wurden die Experimente wie in Beispiel 3 durchgeführt. Nach 5 Minuten Schütteln gebildete Co-Aggregate zwischen Lactobacillus Zellen und Helicobacter pylori-Zellen wurden starken Scherkräften durch Pipettieren der Suspension für 1 Minute oder 2 minütiges Schütteln bei hoher Geschwindigkeit ausgesetzt. Danach wurde die Co-Aggregat-Größe mikroskopisch und makroskopisch untersucht. Die Größe der Co-Aggregate aus sprühgetrockneten Lactobacillus Zellen und Helicobacter pylori nahm nicht ab, wohingegen die Größe der Co-Aggregate aus nicht-sprühgetrockneten Lactobacillus Zellen und Helicobacter pylori abnahm.

### Beispiel 5: Herstellung einer pharmazeutischen Zusammensetzung mit erfindungsgemäßen Lactobacillus Stämmen

Zellen eines Lactobacillus Stammes oder mehrerer Lactobacillus Stämme der Erfindung werden gemäß Beispiel 1 und 2 gezogen und sprühgetrocknet. Das sprühgetrocknete Pulver wird dann auf eine Partikelgröße von maximal ca. 1 mm Durchmesser gemahlen. Das erhaltene Granulat wird in den folgenden Mengenverhältnissen (Gew.-%) mit Träger- bzw. Hilfsstoffen gemischt: 20% Granulat, 2% Siliciumdioxid (Syloid AL-IFP, GRACE Davidson), 1% Magnesiumstearat (MF-2-V, Ackros), 77% mikrokristalline Zellulose (Avicel PH 112, FMC).

Das Mischen erfolgt in einem Quintech Micromixer bei Position 70 Level II. Alle Komponenten werden zugleich zugegeben. Die Mischung erfolgt für ca. 120 s. Anschließend wird die erhaltene Mischung in einer handelsüblichen Tablettenpresse unter üblichen Bedingungen, jedoch möglich niedriger Druckkraft (< 10 kN) zu Tabletten mit einem Gewicht von ca. 500 mg gepresst. Jede Tablette enthält ca. 10⁸ bis 10¹⁰ sprühgetrockneten Lactobacillus Zellen.

### Beschreibung der Figuren:

Figur 1A zeigt Co-Aggregate aus frisch kultivierten nicht-sprühgetrockneten Lactobacillus DSM 17648 Zellen und Helicobacter pylori (Co-Aggregate zeigen eine Größe von 40 µm und mehr) unter simulierten Magenbedingungen.
Figur 1B zeigt ein sehr großes Co-Aggregat aus sprühgetrockneten Lactobacillus DSM 17648 Zellen und Helicobacter pylori (Co-Aggregate zeigen eine Größe von 150 µm und mehr) unter simulierten Magenbedingungen.
Figuren 1C bis 1E dienen als Kontrollen im Experiment.
Figur 1C zeigt frisch kultivierte Lactobacillus DSM 17648 Zellen unter simulierten Magenbedingungen. Eine Autoaggregation ist nicht erkennbar.
Figur 1D zeigt sprühgetrocknete Lactobacillus DSM 17648 Zellen unter simulierten Magenbedingungen. Eine Autoaggregation ist nicht erkennbar.
Figur 1E zeigt frisch kultivierte Helicobacter pylori Zellen unter simulierten Magenbedingungen. Eine Autoaggregation ist nicht erkennbar.
(Figur 1A-E sind 1.000-fach vergrößert)
Figuren 2A und 2B zeigen die Morphologie der Lactobacillus Zellen.
Figur 2A zeigt nicht-sprühgetrocknete Lactobacillus Zellen. Die Zellen liegen in Ketten von 2 bis 10 Zellen vor.
Figur 2B zeigt sprühgetrocknete Lactobacillus Zellen. Die Zellen liegen als Mono- und Dimere vor (1 bis 2 Zellen) und sind gegenüber nicht-sprühgetrockneten Zellen stark verkleinert.

## Patentansprüche

1. Zusammensetzung enthaltend sprühgetrocknete Lactobacillus Zellen zur Behandlung und Prophylaxe von Helicobacter pylori Infektionen an Mensch oder Tier.

2. Zusammensetzung nach Anspruch 1 enthaltend sprühgetrocknete Lactobacillus Zellen, **dadurch gekennzeichnet, dass** diese im Wesentlichen in einer mono und/oder dimeren Form vorliegen.

3. Zusammensetzung nach Anspruch 1 oder 2, wobei nach Applikation im Magenmedium in-situ Co-Aggregate mit Helicobacter pylori gebildet werden.

4. Zusammensetzung nach einem der vorstehenden Ansprüchen, wobei die Co-Aggregate nicht kleiner als 50 µm, insbesondere größer als 500 µm sind.

5. Zusammensetzung nach einem der vorstehenden Ansprüchen, wobei die Lactobacillus Zellen ausgewählt sind aus der Gruppe *Lactobacillus lactis, Lactobacillus helveticus, Lactobacillus acidophilus, Lactobacillus bulgaricus, Lactobacillus delbrueckii, Lactobacillus casei, Lactobacillus paracasei, Lactobacillus pentosus, Lactobacillus rhamnosus* und *Lactobacillus plantarum, Lactobacillus brevis, Lactobacillus fermentum, Lactobacillus viridescens, Bifidobacterium bifidum,* DSM 17646, DSM 17647, 'DSM 17648, DSM 17649, DSM 17650, DSM 17651, DSM 17652, DSM 17653, vorzugsweise *Lactobacillus fermentum, Lactobacillus brevis und Lactobacillus pentosus.*

6. Zusammensetzung nach einem der vorstehenden Ansprüchen, **dadurch gekennzeichnet, dass** eine pharmazeutische oder diätische Zusammensetzung vorliegt, insbesondere in Form eines Nahrungs- bzw. Lebensmittels und/oder eines Futtermittels für Haus-und/oder Nutztiere und / oder Nahrungsergänzungsmittel, ggfs. enthaltend Hilfs- und Zusatzstoffe.

7. Zusammensetzung nach einem der vorstehenden Ansprüchen zur Eradikationstherapie von Helicobacter pylori, ggfs. in Kombination mit weiteren geeigneten Wirkstoffen, wie Antibiotika.

8. Verfahren zur Herstellung einer Zusammensetzung nach Anspruch 1, wobei die sprühgetrockneten Lactobacillus Zellen mit dem physiologisch verträglichen Träger gemischt werden.

9. Verfahren zur Herstellung einer Zusammensetzung nach Anspruch 7, wobei die Lactobacillus Zellen zuerst (i) optional angeimpft und angereichert, (ii) fermentiert und (iii) sprühgetrocknet werden.

10. Verwendung von sprühgetrockneten Lactobacillus Zellen zur Herstellung einer pharmazeutischen oder dietätischen Zusammensetzung, insbesondere zur Prophylaxe und/oder Behandlung von durch Infektion mit Helicobacter pylori bedingten Erkrankungen, insbesondere Gastrointestinalerkrankungen, wie Gastritis, Magengeschwüre, Ulcus und Magenkrebs.

11. Verwendung von sprühgetrockneten Lactobacillus Zellen zur Eradikationstherapie von Helicobacter pylori, ggfs. in Kombination mit weiteren geeigneten Wirkstoffen, wie Antibiotika.
